# EUROPEAN PATENT APPLICATION

(11) **EP 1 437 364 A2**
(43) Date of publication of application: **14.07.2004**
(21) Application number: 03028628.0
(22) Date of filing: 15.12.2003
(51) Int. Cl.: C07K 14/47

(54) **Polypeptide binding to the androgen receptor and its potential use for treating breast cancer**

(30) Priority: 16.12.2002 US 320203
(71) Applicant: TargetGen, Inc., Taipei 110 (TW)
(72) Inventor: Chang, Tay-Jay, Taipei (TW)
(74) Representative: Reinhard - Skuhra - Weise & Partner GbR

(57) **Abstract**

New androgen receptor down-regulatory-associated proteins (i.e., ARDAPs) that bind to and reduce the transactivation activity of androgen receptors, and nucleic acids encoding them.

SEQUENCE LISTING

<110> TargetGen, Inc.

<120> TREATING BREAST CANCER

<130> P16726

<140> EP
<141> 2003-12-15

<150> US 10/320,203
<151> 2002-12-16

<160> 4

<170> FastSEQ for Windows Version 4.0

<210> 1
<211> 225
<212> PRT
<213> Homo sapiens

<400> 1
Met Ala Gly Thr Gly Leu Val Ala Gly Glu Val Val Val Asp Ala Leu
 1               5                  10                  15
Pro Tyr Phe Asp Gln Gly Tyr Glu Ala Pro Gly Val Arg Glu Ala Ala
            20                  25                  30
Ala Ala Leu Val Glu Glu Glu Thr Arg Arg Tyr Arg Pro Thr Lys Asn
        35                  40                  45
Tyr Leu Ser Tyr Leu Thr Ala Pro Asp Tyr Ser Ala Phe Glu Thr Asp
    50                  55                  60
Ile Met Arg Asn Glu Phe Glu Arg Leu Ala Ala Arg Gln Pro Ile Glu
65                  70                  75                  80
Leu Leu Ser Met Lys Arg Tyr Glu Leu Pro Ala Pro Ser Ser Gly Gln
                85                  90                  95
Lys Asn Asp Ile Thr Ala Trp Gln Glu Cys Val Asn Asn Ser Met Ala
            100                 105                 110
Gln Leu Glu His Gln Ala Val Arg Ile Glu Asn Leu Glu Leu Met Ser
        115                 120                 125
Gln His Gly Cys Asn Ala Trp Lys Val Tyr Asn Glu Asn Leu Val His
    130                 135                 140
Met Ile Glu His Ala Gln Lys Glu Leu Gln Lys Leu Arg Lys His Ile
145                 150                 155                 160
Gln Asp Leu Asn Trp Gln Arg Lys Asn Met Gln Leu Thr Ala Gly Ser
                165                 170                 175
Lys Leu Arg Glu Met Glu Ser Asn Trp Val Ser Leu Val Ser Lys Asn
            180                 185                 190
Tyr Glu Ile Glu Arg Thr Ile Val Gln Leu Glu Asn Glu Ile Tyr Gln
        195                 200                 205
Ile Lys Gln Gln His Gly Glu Ala Asn Lys Glu Asn Ile Arg Gln Asp
    210                 215                 220
Phe
225

<210> 2
<211> 678
<212> DNA
<213> Homo sapiens

<220>
<221> CDS
<222> (1)...(675)

<400> 2
atg gcg ggc aca ggt ttg gtg gct gga gag gtt gtg gtg gat gcg ctg      48
Met Ala Gly Thr Gly Leu Val Ala Gly Glu Val Val Val Asp Ala Leu
 1               5                  10                  15

ccg tat ttt gat caa ggt tat gaa gcc cct ggt gtg cgg gaa gcg gct      96
Pro Tyr Phe Asp Gln Gly Tyr Glu Ala Pro Gly Val Arg Glu Ala Ala
            20                  25                  30

gca gcg ctg gtg gag gag gaa act cgc aga tac cga cct act aag aac     144
Ala Ala Leu Val Glu Glu Glu Thr Arg Arg Tyr Arg Pro Thr Lys Asn
        35                  40                  45

tac ctg agc tac ctg aca gcc ccg gat tat tct gcc ttt gaa act gac     192
Tyr Leu Ser Tyr Leu Thr Ala Pro Asp Tyr Ser Ala Phe Glu Thr Asp
    50                  55                  60

ata atg aga aat gaa ttt gaa aga ctg gct gct cga caa cca att gaa     240
Ile Met Arg Asn Glu Phe Glu Arg Leu Ala Ala Arg Gln Pro Ile Glu
 65                  70                  75                  80

ttg ctc agt atg aaa cga tat gag ctt cca gcc ccc tcc tct ggt caa     288
Leu Leu Ser Met Lys Arg Tyr Glu Leu Pro Ala Pro Ser Ser Gly Gln
                 85                  90                  95

aaa aat gac att act gca tgg caa gaa tgt gta aac aat tct atg gcc     336
Lys Asn Asp Ile Thr Ala Trp Gln Glu Cys Val Asn Asn Ser Met Ala
            100                 105                 110

cag tta gag cat caa gca gtt aga att gag aat ctg gaa cta atg tca     384
Gln Leu Glu His Gln Ala Val Arg Ile Glu Asn Leu Glu Leu Met Ser
        115                 120                 125

cag cat gga tgt aat gcc tgg aaa gta tac aat gaa aat cta gtt cat     432
Gln His Gly Cys Asn Ala Trp Lys Val Tyr Asn Glu Asn Leu Val His
    130                 135                 140

atg att gaa cac gca cag aag gaa ctt cag aag tta aga aaa cat att     480
Met Ile Glu His Ala Gln Lys Glu Leu Gln Lys Leu Arg Lys His Ile
145                 150                 155                 160

caa gat tta aac tgg cag aga aag aac atg caa ctc aca gct gga tct     528
Gln Asp Leu Asn Trp Gln Arg Lys Asn Met Gln Leu Thr Ala Gly Ser
                165                 170                 175

aaa ttg aga gaa atg gag tca aat tgg gta tcc ctg gtc agt aag aat     576
Lys Leu Arg Glu Met Glu Ser Asn Trp Val Ser Leu Val Ser Lys Asn

180                 185                 190

tat gag att gaa cgg act att gtt cag cta gaa aat gaa atc tat caa       624
Tyr Glu Ile Glu Arg Thr Ile Val Gln Leu Glu Asn Glu Ile Tyr Gln
        195                 200                 205

att aag cag caa cat gga gag gca aac aaa gaa aac atc cgg caa gac       672
Ile Lys Gln Gln His Gly Glu Ala Asn Lys Glu Asn Ile Arg Gln Asp
    210                 215                 220

ttc taa                                                               678
Phe
225


<210> 3
<211> 678
<212> DNA
<213> Mus musculus

<220>
<221> CDS
<222> (1)...(675)

<400> 3
atg gcg ggc acg ggc ttg gta gcc gga gag gtg gtg gta gat gcg cta        48
Met Ala Gly Thr Gly Leu Val Ala Gly Glu Val Val Val Asp Ala Leu
 1               5                  10                  15

cca tat ttt gac caa ggc tat gag gca ccc ggc gtg cgg gaa gcg gcg        96
Pro Tyr Phe Asp Gln Gly Tyr Glu Ala Pro Gly Val Arg Glu Ala Ala
            20                  25                  30

gcg gcc ctg gtg gag gag gag acg cgc aga tac cga cct acc aag aac       144
Ala Ala Leu Val Glu Glu Glu Thr Arg Arg Tyr Arg Pro Thr Lys Asn
        35                  40                  45

tac ctg agc tac ctg acg gcc ccg gat tat tct gcc ttt gaa aca gac       192
Tyr Leu Ser Tyr Leu Thr Ala Pro Asp Tyr Ser Ala Phe Glu Thr Asp
    50                  55                  60

ata atg aga aat gaa ttt gaa aga ctc gct gct cga caa ccg att gaa       240
Ile Met Arg Asn Glu Phe Glu Arg Leu Ala Ala Arg Gln Pro Ile Glu
65                  70                  75                  80

tta ctc agc atg aaa cga tat gaa ctt cca gcc cct tcc tca ggt caa       288
Leu Leu Ser Met Lys Arg Tyr Glu Leu Pro Ala Pro Ser Ser Gly Gln
                85                  90                  95

aaa aat gac att act gca tgg caa gaa tgt gta aac aat tct atg gct       336
Lys Asn Asp Ile Thr Ala Trp Gln Glu Cys Val Asn Asn Ser Met Ala
            100                 105                 110

cag ttg gag cac cag gcg gtc cgg atc gag aat ctg gag ctg atg tca       384
Gln Leu Glu His Gln Ala Val Arg Ile Glu Asn Leu Glu Leu Met Ser
        115                 120                 125

cag cat gga tgc aat gcc tgg aag gtg tac aat gaa aat ctt gtt cat 432
Gln His Gly Cys Asn Ala Trp Lys Val Tyr Asn Glu Asn Leu Val His
    130                 135                 140

atg att gaa cat gca cag aaa gag ctt cag aag tta agg aaa cat att 480
Met Ile Glu His Ala Gln Lys Glu Leu Gln Lys Leu Arg Lys His Ile
145                 150                 155                 160

caa gat ttg aac tgg cag cga aag aac atg cag ctt aca gct gga tct 528
Gln Asp Leu Asn Trp Gln Arg Lys Asn Met Gln Leu Thr Ala Gly Ser
                165                 170                 175

aag ctg aga gaa atg gag tca aac tgg gtg tcg ctg gtg agt aag aac 576
Lys Leu Arg Glu Met Glu Ser Asn Trp Val Ser Leu Val Ser Lys Asn
            180                 185                 190

tat gag att gag cgg acg att gtc cag ctg gag aac gag atc tat cag 624
Tyr Glu Ile Glu Arg Thr Ile Val Gln Leu Glu Asn Glu Ile Tyr Gln
        195                 200                 205

atc aag cag cag cac ggg gag gcc aac aag gaa aac atc cgc caa gac 672
Ile Lys Gln Gln His Gly Glu Ala Asn Lys Glu Asn Ile Arg Gln Asp
    210                 215                 220

ttc taa 678
Phe
225

<210> 4
<211> 225
<212> PRT
<213> Mus musculus

<400> 4
Met Ala Gly Thr Gly Leu Val Ala Gly Glu Val Val Val Asp Ala Leu
1               5                   10                  15
Pro Tyr Phe Asp Gln Gly Tyr Glu Ala Pro Gly Val Arg Glu Ala Ala
            20                  25                  30
Ala Ala Leu Val Glu Glu Glu Thr Arg Arg Tyr Arg Pro Thr Lys Asn
        35                  40                  45
Tyr Leu Ser Tyr Leu Thr Ala Pro Asp Tyr Ser Ala Phe Glu Thr Asp
    50                  55                  60
Ile Met Arg Asn Glu Phe Glu Arg Leu Ala Ala Arg Gln Pro Ile Glu
65                  70                  75                  80
Leu Leu Ser Met Lys Arg Tyr Glu Leu Pro Ala Pro Ser Ser Gly Gln
                85                  90                  95
Lys Asn Asp Ile Thr Ala Trp Gln Glu Cys Val Asn Asn Ser Met Ala
            100                 105                 110
Gln Leu Glu His Gln Ala Val Arg Ile Glu Asn Leu Glu Leu Met Ser
        115                 120                 125
Gln His Gly Cys Asn Ala Trp Lys Val Tyr Asn Glu Asn Leu Val His
    130                 135                 140
Met Ile Glu His Ala Gln Lys Glu Leu Gln Lys Leu Arg Lys His Ile
145                 150                 155                 160
Gln Asp Leu Asn Trp Gln Arg Lys Asn Met Gln Leu Thr Ala Gly Ser
                165                 170                 175

Lys Leu Arg Glu Met Glu Ser Asn Trp Val Ser Leu Val Ser Lys Asn
            180                 185                 190
Tyr Glu Ile Glu Arg Thr Ile Val Gln Leu Glu Asn Glu Ile Tyr Gln
        195                 200                 205
Ile Lys Gln Gln His Gly Glu Ala Asn Lys Glu Asn Ile Arg Gln Asp
    210                 215                 220
Phe
225

## Description

### BACKGROUND

A variety of genes that are overexpressed in tumor cells relative to healthy cells have been identified. It is expected that the identification of such genes will provide drug targets for anti-cancer drug development and for cancer diagnostics.

Steroid hormones generally exert their physiological effects by binding to their specific nuclear receptors to form complexes that in turn act as transcriptional factors. The complexes bind to specific nucleotide sequences (i.e., steroid responsive elements) in the promoters of steroid-responsive genes to facilitate transcription of those genes.

### SUMMARY

The invention is based on the discovery of a human protein that is overexpressed in breast cancer cells (relative to adjacent normal cells), and binds to an androgen receptor (AR) and reduces the ability of the receptor to transactivate an androgen-responsive gene. Thus, this newly discovered human protein is named "androgen receptor down-regulatory-associated protein" or "ARDAP." The human ARDAP amino acid sequence (SEQ ID NO:1) and the nucleic acid sequence encoding it (SEQ ID NO:2) are shown below:

1/1                                    31/11
atg gcg ggc aca ggt ttg gtg gct gga gag gtt gtg gtg gat gcg ctg ccg tat ttt gat

61/21                                  91/31
caa ggt tat gaa gcc cct ggt gtg cgg gaa gcg gct gca gcg ctg gtg gag gag gaa act

121/41                                 151/51
cgc aga tac cga cct act aag aac tac ctg agc tac ctg aca gcc ccg gat tat tct gcc

181/61                                 211/71
ttt gaa act gac ata atg aga aat gaa ttt gaa aga ctg gct gct cga caa cca att gaa

241/81                                 271/91
ttg ctc agt atg aaa cga tat gag ctt cca gcc ccc tcc tct ggt caa aaa aat gac att

301/101                                331/111
act gca tgg caa gaa tgt gta aac aat tct atg gcc cag tta gag cat caa gca gtt aga

361/121                                391/131
att gag aat ctg gaa cta atg tca cag cat gga tgt aat gcc tgg aaa gta tac aat gaa

421/141                                451/151
aat cta gtt cat atg att gaa cac gca cag aag gaa ctt cag aag tta aga aaa cat att

asn leu val his met ile glu his ala gln lys glu leu gln lys leu arg lys his ile
481/161                                 511/171
caa gat tta aac tgg cag aga aag aac atg caa ctc aca gct gga tct aaa ttg aga gaa
gln asp leu asn trp gln arg lys asn met gln leu thr ala gly ser lys leu arg glu
541/181                                 571/191
atg gag tca aat tgg gta tcc ctg gtc agt aag aat tat gag att gaa cgg act att gtt
met glu ser asn trp val ser leu val ser lys asn tyr glu ile glu arg thr ile val
601/201                                 631/211
cag cta gaa aat gaa atc tat caa att aag cag caa cat gga gag gca aac aaa gaa aac
gln leu glu asn glu ile tyr gln ile lys gln gln his gly glu ala asn lys glu asn
661/221
atc cgg caa gac ttc tga (SEQ ID NO:2)
ile arg gln asp phe (SEQ ID NO:1)

A mouse ARDAP gene (SEQ ID NO:3) has also been cloned. It encodes a mouse ARDAP protein (SEQ ID NO:4) that is 90% identical to the human ARDAP protein.

1/1                                     31/11
atg gcg ggc acg ggc ttg gta gcc gga gag gtg gtg gta gat gcg cta cca tat ttt gac
met ala gly thr gly leu val ala gly glu val val val asp ala leu pro tyr phe asp
61/21                                   91/31
caa ggc tat gag gca ccc ggc gtg cgg gaa gcg gcg gcg gcc ctg gtg gag gag gag acg
gln gly tyr glu ala pro gly val arg glu ala ala ala ala leu val glu glu glu thr
121/41                                  151/51
cgc aga tac cga cct acc aag aac tac ctg agc tac ctg acg gcc ccg gat tat tct gcc
arg arg tyr arg pro thr lys asn tyr leu ser tyr leu thr ala pro asp tyr ser ala
181/61                                  211/71
ttt gaa aca gac ata atg aga aat gaa ttt gaa aga ctc gct gct cga caa ccg att gaa
phe glu thr asp ile met arg asn glu phe glu arg leu ala ala arg gln pro ile glu
241/81                                  271/91
tta ctc agc atg aaa cga tat gaa ctt cca gcc cct tcc tca ggt caa aaa aat gac att
leu leu ser met lys arg tyr glu leu pro ala pro ser ser gly gln lys asn asp ile
301/101                                 331/111
act gca tgg caa gaa tgt gta aac aat tct atg gct cag ttg gag cac cag gcg gtc cgg
thr ala trp gln glu cys val asn asn ser met ala gln leu glu his gln ala val arg
361/121                                 391/131
atc gag aat ctg gag ctg atg tca cag cat gga tgc aat gcc tgg aag gtg tac aat gaa
ile glu asn leu glu leu met ser gln his gly cys asn ala trp lys val tyr asn glu
421/141                                 451/151
aat ctt gtt cat atg att gaa cat gca cag aaa gag ctt cag aag tta agg aaa cat att
asn leu val his met ile glu his ala gln lys glu leu gln lys leu arg lys his ile
481/161                                 511/171
caa gat ttg aac tgg cag cga aag aac atg cag ctt aca gct gga tct aag ctg aga gaa
gln asp leu asn trp gln arg lys asn met gln leu thr ala gly ser lys leu arg glu
541/181                                 571/191
atg gag tca aac tgg gtg tcg ctg gtg agt aag aac tat gag att gag cgg acg att gtc
met glu ser asn trp val ser leu val ser lys asn tyr glu ile glu arg thr ile val
601/201                                 631/211
cag ctg gag aac gag atc tat cag atc aag cag cag cac ggg gag gcc aac aag gaa aac
gln leu glu asn glu ile tyr gln ile lys gln gln his gly glu ala asn lys glu asn
661/221
atc cgc caa gac ttc taa (SEQ ID NO:3)
ile arg gln asp phe (SEQ ID NO:4)

Accordingly, the invention features a pure polypeptide including an amino acid sequence at least 70% (e.g., at least 75, 80, 85, 90, 95, or 99; or 100%) identical to SEQ ID NO:1 or 4. When expressed in a cell, the polypeptide binds to an AR and decreases its transactivation activity. The polypeptides of the invention can be used for producing ARDAP antibodies (either monoclonal or polyclonal). These antibodies in turn are useful for detecting the presence and distribution of ARDAP proteins in tissues and in cellular compartments. For example, such antibodies can be used to verify the expression of ARDAP proteins in a transgenic animal.

A "pure polypeptide" refers to a polypeptide substantially free from naturally associated molecules, i.e., it is at least 75% (e.g., at least 80, 85, 90, or 95; or 100%) pure by dry weight. Purity can be measured by any appropriate standard method, for example, by column chromatography, polyacrylamide gel electrophoresis, or HPLC analysis.

The "percent identity" of two amino acid sequences is determined using the algorithm of Karlin and Altschul ((1990) Proc. Natl. Acad. Sci. USA 87, 2264-2268), modified as in Karlin and Altschul ((1993) Proc. Natl. Acad. Sci. USA 90, 5873-5877). Such an algorithm is incorporated into the XBLAST programs of Altschul, et al. ((1990) J. Mol. Biol. 215, 403-410). BLAST protein searches are performed with the XBLAST program, score = 50, wordlength = 3. Where gaps exist between two sequences, Gapped BLAST is utilized as described in Altschul, et al. ((1997) Nucleic Acids Res. 25, 3389-3402). When utilizing BLAST and Gapped BLAST programs, the default parameters of the respective programs (e.g., XBLAST) are used. See www.ncbi.nlm.nih.gov.

The invention further features an isolated nucleic acid characterized in that it hybridizes under stringent conditions to SEQ ID NO:2 or 3, or a complementary sequence thereof, as well as a cell (in a culture or in a transgenic animal) containing a nucleic acid of the invention. Such a nucleic acid can be at least 15 (e.g., at least 30, 50, 100, 200, 500, or 1000) nucleotides in length. An example of a nucleic acid within the invention is an isolated nucleic acid (e.g., a vector) encoding a polypeptide of the invention, e.g., a nucleic acid that contains SEQ ID NO: 2 or 3. These nucleic acids and cells can be used for producing the polypeptides of the invention or generating a transgenic animal. For example, the nucleic acids of the invention can be used to determine whether an ARDAP mRNA is expressed in a tissue or cell. The nucleic acids can be used as primers in PCR-based detection methods, or as labeled probes in nucleic acid blots (e.g., Northern blots).

An "isolated nucleic acid" is a nucleic acid the structure of which is not identical to that of any naturally occurring nucleic acid or to that of any fragment of a naturally occurring genomic nucleic acid. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein.

By hybridization under "stringent conditions" is meant hybridization at 65°C, 0.5 X SSC, followed by washing at 45°C, 0.1 X SSC.

In addition, the invention features a method of (1) expressing in a cell (e.g., a breast cell) a transcript, i.e., transcript I, that hybridizes under above-described stringent conditions to SEQ ID NO:2 or 3, or (2) expressing in a cell (e.g., a breast cell) a transcript, i.e., transcript II, that is complementary to transcript I. Transcript I, when expressed in a cell, can serve as an anti-sense RNA that binds to endogenous ARDAP mRNA to prevent it from being translated into a functional protein. Therefore, this method can be used in gene therapy for treating cancer (e.g., breast cancer). Transcript II can encode an ARDAP protein, and when expressed in a cell, is translated into an ARDAP protein. Thus, this method can be used for producing a polypeptide of the invention.

ARDAP has been found to be expressed in breast cancer but not in normal breast tissue. It is thus useful for diagnosing and treating cancer (e.g., breast cancer).

In one aspect, this invention features a method of determining whether a subject (i.e., an animal or a human) is suffering from or at risk for developing breast cancer. In one example, the method includes providing a sample (e.g., a breast sample) from a subject and determining the ARDAP expression level in the sample. If the ARDAP expression level in the sample is higher than that in a sample from a normal subject, it indicates that the subject is suffering from or at risk for developing breast cancer. The ARDAP expression level can be determined by measuring the amount of the ARDAP mRNA or the ARDAP protein. The ARDAP mRNA level can be determined, for example, by in situ hybridization, PCR, or Northern blot analysis. The ARDAP protein level can be determined, for example, by Western blot analysis. In another example, the method includes providing a sample (e.g., a breast sample) from a subject and determining the ARDAP activity level in the sample. If the ARDAP activity level in the sample is higher than that in a sample from a normal subject, it indicates that the subject is suffering from or at risk for developing breast cancer. The ARDAP activity level can be determined by measuring its ability to bind AR or to inhibit the transactivation activity ofAR.

In another aspect, this invention features a method of identifying a compound for treating breast cancer. The method includes contacting a compound with a cell (e.g., a breast cell) and determining the ARDAP expression or activity level in the cell. If the ARDAP expression or activity level in the presence of the compound is lower than that in the absence of the compound, the compound is a candidate for treating breast cancer.

In still another aspect, this invention features a method of treating breast cancer. The method includes identifying a subject (i.e., an animal or a human) suffering from or being at risk for developing breast cancer and administering to the subject a composition to decrease the ARDAP expression or activity level in the subject. The composition can contain a nucleic acid encoding a transcript characterized in that it hybridizes under stringent conditions to the sense strand of the ARDAP gene. This transcript, when expressed in a cell (e.g., a breast cell), can serve as an anti-sense RNA that binds to endogenous ARDAP mRNA to prevent it from being translated into a functional protein. Therefore, this method can be used in gene therapy for treating breast cancer. The activity of an ARDAP protein can be inhibited by an ARDAP antibody (e.g., delivered to a breast cell).

Also within the scope of this invention is a pharmaceutical composition for treating breast cancer. In one example, the composition contains a pharmaceutically acceptable carrier and a nucleic acid encoding a transcript characterized in that it hybridizes under stringent conditions to the sense strand of the ARDAP gene. In another example, the composition contains a pharmaceutically acceptable carrier and an ARDAP antibody.

The invention also features a transgenic animal (e.g., a rodent such as a mouse) whose genome includes a transgene containing a nucleic acid of this invention and that exhibits a decreased androgen response as compared to a wild-type animal. The transgenic animal can be generated by introducing a nucleic acid of the invention into a cell such that a transcript is produced from the nucleic acid and the transcript is translated into a protein. These transgenic animals can be used as models for developing drugs to treat cancer (e.g., breast cancer).

This invention is also based on the unexpected finding that the number of androgen receptors (ARs) in breast tumor cells decreases relative to their adjacent healthy breast cells.

Accordingly, in one aspect, this invention features a method of determining whether a subject is suffering from or at risk for developing breast cancer. In one example, the method includes providing a sample (e.g., a breast sample) from a subject and determining the androgen receptor gene expression level in the sample. If the androgen receptor gene expression level in the sample is lower than that in a sample from a normal subject, it indicates that the subject is suffering from or at risk for developing breast cancer. The androgen receptor gene expression level can be determined by measuring the amount of the androgen receptor mRNA, or the androgen receptor protein. The androgen receptor mRNA level can be determined, for example, by in situ hybridization, PCR, or Northern blot analysis. The androgen receptor protein level can be determined, for example, by Western blot analysis. In another example, the method includes providing a sample from a subject and determining the androgen receptor protein activity level in the sample. If the androgen receptor protein activity level in the sample is lower than that in a sample from a normal subject, it indicates that the subject is suffering from or at risk for developing breast cancer. The androgen receptor protein activity can be determined, e.g., by measuring its ability to transactivate the expression of an androgen-responsive gene.

In another aspect, this invention features a method of identifying a compound for treating breast cancer. In one example, the method includes contacting a compound with a cell (e.g., a breast cell) and determining the androgen receptor gene expression level in the cell. If the androgen receptor gene expression level in the presence of the compound is higher than that in the absence of the compound, the compound is a candidate for treating breast cancer. In another example, the method includes contacting a compound with a cell and determining the androgen receptor protein activity level in the cell. If the androgen receptor protein activity level in the presence of the compound is higher than that in the absence of the compound, the compound is a candidate for treating breast cancer.

In still another aspect, this invention features a method of treating breast cancer. In one example, the method includes identifying a subject suffering from or being at risk for developing breast cancer and administering to the subject a composition to increase the androgen receptor level in the subject. The composition can contain a nucleic acid encoding an androgen receptor protein or an androgen receptor itself. An "androgen receptor protein" refers to both a wild-type androgen receptor protein and its variants with an equivalent biological function (e.g., a fragment of a wild-type androgen receptor protein). The composition can be administered directly to a breast cell in the subject. In another example, the method includes identifying a subject suffering from or being at risk for developing breast cancer and administering to the subject a composition to increase the androgen receptor activity level in the subject.

The details of one or more embodiments of the invention are set forth in the accompanying description below. Other features, objects, and advantages of the invention will be apparent from the detailed description, and from the claims.

### DETAILED DESCRIPTION

The invention relates to new ARDAP proteins and nucleic acids encoding them that are overexpressed in breast cancer cells relative to normal breast cells. Unexpectedly, ARDAP was found to bind to and reduce the transactivation activity of an AR. These observations suggest that ARDAP inhibits, via an AR, mitogenic genes that are androgen-responsive (i.e., genes whose promoters contain androgen responsive elements), that overexpression of ARDAP leads to cancer by reducing AR-mediated transactivation of androgen-responsive mitogenic genes, and that inhibition of ARDAP expression or activity would increase expression of these androgen-responsive mitogenic genes and revert cancer cells to a more normal phenotype. Consequently, ARDAP is a new cancer drug target.

In one aspect, the present invention features pure ARDAP polypeptides (e.g., SEQ ID NO:1), including functional ARDAP polypeptides. A "functional polypeptide" refers to a polypeptide which possesses biological activity equivalent to that of a wild-type ARDAP protein, e.g., a fragment of a wild-type ARDAP protein.

In another aspect, the invention features isolated ARDAP nucleic acids (i.e., DNA, cDNA, and RNA) characterized in that they hybridize under stringent conditions to SEQ ID NO:2 or 3, or a complementary sequence thereof. The nucleic acids of the invention include sequences that are degenerate as a result of the genetic code.

A nucleic acid of the invention can be expressed in vitro by DNA transfer into a suitable host cell by methods known in the art. For example, the nucleic acid can be inserted into a recombinant expression vector. A variety of host-expression vector systems can be utilized to express a nucleic acid of the invention. These include, but are not limited to, microorganisms such as bacteria transformed with recombinant bacteriophage DNA, plasmid DNA, or cosmid DNA expression vectors; yeast transformed with recombinant yeast expression vectors; and human cell lines infected with recombinant virus or plasmid expression vectors. Isolation and purification of recombinant polypeptides, or fragments thereof, provided by the invention, can be carried out by conventional means including preparative chromatography and immunological separations involving monoclonal or polyclonal antibodies.

The invention also features antibodies against the ARDAP polypeptide, including monoclonal antibodies and polyclonal antibodies. The term "antibody" includes intact molecules as well as fragments thereof, such as Fab, F(ab')₂, and Fv which are capable of binding to an epitopic determinant present in the ARDAP polypeptide. Methods of making monoclonal and polyclonal antibodies and fragments thereof are known in the art. See, for example, Harlow and Lane, (1988) Antibodies: A Laboratory Manual, Cold Spring Harbor Laboratory, New York. The ARDAP antibodies can be used to inhibit ARDAP protein activity, and thus useful for preventing and treating cancer (e.g., breast cancer).

In addition, the invention provides methods for diagnosing and treating cancer (e.g., breast cancer), and identifying therapeutic compounds for treating such a disease using ARDAP nucleic acids and polypeptides.

A diagnostic method of this invention involves comparing the ARDAP gene expression level or the ARDAP protein activity level in a sample (e.g., a breast sample) prepared from a subject (i.e., an animal or a human) with that in a sample prepared from a normal subject, i.e., a subject who does not suffer from breast cancer. A higher ARDAP gene expression level or ARDAP protein activity level indicates that the subject is suffering from or at risk for developing breast cancer. The methods of this invention can be used on their own or in conjunction with other procedures to diagnose breast cancer in appropriate subjects.

The ARDAP gene expression level can be determined at either the mRNA level or at the protein level. Methods of measuring mRNA levels in a tissue sample are known in the art. In order to measure mRNA levels, cells can be lysed and the levels of ARDAP mRNA in the lysates or in RNA purified or semi-purified from the lysates can be determined by any of a variety of methods including, without limitation, hybridization assays using detectably labeled ARDAP-specific DNA or RNA probes and quantitative or semi-quantitative RT-PCR methodologies using appropriate ARDAP-specific oligonucleotide primers. Alternatively, quantitative or semi-quantitative in situ hybridization assays can be carried out using, for example, tissue sections or unlysed cell suspensions, and detectably (e.g., fluorescently or enzyme) labeled DNA or RNA probes. Additional methods for quantifying mRNA include RNA protection assay (RPA) and SAGE.

Methods of measuring protein levels in a tissue sample are also known in the art. Many such methods employ antibodies (e.g., monoclonal or polyclonal antibodies) that bind specifically to the ARDAP protein. In such assays, the antibody itself or a secondary antibody that binds to it can be detectably labeled. Alternatively, the antibody can be conjugated with biotin, and detectably labeled avidin (a polypeptide that binds to biotin) can be used to detect the presence of the biotinylated antibody. Combinations of these approaches (including "multi-layer sandwich" assays) familiar to those in the art can be used to enhance the sensitivity of the methodologies. Some of these protein-measuring assays (e.g., ELISA or Western blot) can be applied to lysates of cells, and others (e.g., immunohistological methods or fluorescence flow cytometry) applied to histological sections or unlysed cell suspensions. Methods of measuring the amount of label will be depend on the nature of the label and are well known in the art. Appropriate labels include, without limitation, radionuclides (e.g., ¹²⁵I, ¹³¹I, ³⁵S, ³H, or ³²P), enzymes (e.g., alkaline phosphatase, horseradish peroxidase, luciferase, or β-glactosidase), fluorescent moieties or proteins (e.g., fluorescein, rhodamine, phycoerythrin, GFP, or BFP), or luminescent moieties (e.g., Qdot™ nanoparticles supplied by the Quantum Dot Corporation, Palo Alto, CA). Other applicable assays include quantitative immunoprecipitation or complement fixation assays.

The ARDAP protein activity level can be determined by measuring its ability to bind the AR or to inhibit the transactivation activity of the AR. See, e.g., the Example below.

The invention also provides a method for identifying and manufacturing compounds (e.g., proteins, peptides, peptidomimetics, peptoids, antibodies, or small molecules) that decrease the ARDAP gene expression level or ARDAP protein activity level in a cell (e.g., a breast cell). Compounds thus identified can be used, e.g., for preventing and treating breast cancer.

The candidate compounds of the present invention can be obtained using any of the numerous approaches in combinatorial library methods known in the art. Such libraries include: peptide libraries, peptoid libraries (libraries of molecules having the functionalities of peptides, but with a novel, non-peptide backbone that is resistant to enzymatic degradation); spatially addressable parallel solid phase or solution phase libraries; synthetic libraries obtained by deconvolution or affinity chromatography selection; and the "one-bead one-compound" libraries. See, e.g., Zuckermann et al. (1994) J. Med. Chem. 37, 2678-85; and Lam (1997) Anticancer Drug Des. 12, 145.

Examples of methods for the synthesis of molecular libraries can be found in the art, for example, in: DeWitt et al. (1993) PNAS USA 90, 6909, Erb et al. (1994) PNAS USA 91, 11422; Zuckermann et al. (1994) J. Med. Chem. 37, 2678; Cho et al. (1993) Science 261, 1303; Carrell et al. (1994) Angew. Chem. Int. Ed. Engl. 33, 2059; Carell et al. (1994) Angew. Chem. Int. Ed. Engl. 33, 2061; and Gallop et al. (1994) J. Med. Chem. 37,1233. Libraries of compounds may be presented in solution (e.g., Houghten (1992) Biotechniques 13, 412-421), or on beads (Lam (1991) Nature 354, 82-84), chips (Fodor (1993) Nature 364, 555-556), bacteria (Ladner, U.S. Patent No. 5,223,409), spores (Ladner, U.S. Patent No. 5,223,409), plasmids (Cull et al. (1992) PNAS USA 89, 1865-1869), or phages (Scott and Smith (1990) Science 249, 386-390; Devlin (1990) Science 249, 404-406; Cwirla et al. (1990) PNAS USA 87, 6378-6382; Felici (1991) J. Mol. Biol. 222, 301-310; and Ladner supra).

To identify compounds that decrease the ARDAP gene expression level or ARDAP protein activity level in a cell, a cell (e.g., a breast cell) is contacted with a candidate compound and the ARDAP gene expression level or ARDAP protein activity level is evaluated relative to that in the absence of the candidate compound. The cell can be a cell that naturally expresses ARDAP, or a cell that is modified to express a recombinant nucleic acid, for example, having the ARDAP promoter fused to a marker gene. The ARDAP gene expression level, the marker gene expression level, the ARDAP protein activity level, or the marker protein activity level can be determined by methods described above and any other methods well known in the art. If the ARDAP gene expression level, the marker gene expression level, the ARDAP protein activity level, or the marker protein activity level is lower in the presence of the candidate compound than that in the absence of the candidate compound, the candidate compound is identified as being useful for preventing and treating breast cancer.

This invention also provides a method for preventing and treating breast cancer. Subjects to be treated can be identified, for example, by determining the ARDAP gene expression level or ARDAP protein activity level in a sample prepared from a subject by methods described above. If the ARDAP gene expression level or ARDAP protein activity level is higher in the sample from the subject than that in a sample from a normal subject, the subject is a candidate for treatment with an effective amount of compound that decreases the ARDAP gene expression level or ARDAP protein activity level in the subject. This method can be performed alone or in conjunction with other drugs or therapy.

In one in vivo approach, a therapeutic composition (e.g., a composition containing a compound that decreases the ARDAP gene expression level or ARDAP protein activity level in a cell) is administered to the subject. Generally, the compound will be suspended in a pharmaceutically-acceptable carrier (e.g., physiological saline) and administered orally or by intravenous infusion, or injected or implanted subcutaneously, intramuscularly, intrathecally, intraperitoneally, intrarectally, intravaginally, intranasally, intragastrically, intratracheally, or intrapulmonarily. For prevention and treatment of breast cancer, the compound can be delivered directly to the breast tissue.

The dosage required depends on the choice of the route of administration; the nature of the formulation; the nature of the subject's illness; the subject's size, weight, surface area, age, and sex; other drugs being administered; and the judgment of the attending physician. Suitable dosages are in the range of 0.01-100.0 µg/kg. Wide variations in the needed dosage are to be expected in view of the variety of compounds available and the different efficiencies of various routes of administration. For example, oral administration would be expected to require higher dosages than administration by i.v. injection. Variations in these dosage levels can be adjusted using standard empirical routines for optimization as is well understood in the art. Encapsulation of the compound in a suitable delivery vehicle (e.g., polymeric microparticles or implantable devices) may increase the efficiency of delivery, particularly for oral delivery.

Alternatively, a polynucleotide containing a nucleic acid sequence encoding an anti-sense ARDAP RNA can be delivered to the subject, for example, by the use of polymeric, biodegradable microparticle or microcapsule delivery devices known in the art.

Another way to achieve uptake of the nucleic acid is using liposomes, prepared by standard methods. The vectors can be incorporated alone into these delivery vehicles or co-incorporated with tissue-specific antibodies. Alternatively, one can prepare a molecular conjugate composed of a plasmid or other vector attached to poly-L-lysine by electrostatic or covalent forces. Poly-L-lysine binds to a ligand that can bind to a receptor on target cells (Cristiano et al. (1995) J. Mol. Med. 73, 479). Alternatively, tissue specific targeting can be achieved by the use of tissue-specific transcriptional regulatory elements (TRE) which are known in the art. Delivery of "naked DNA" (i.e., without a delivery vehicle) to an intramuscular, intradermal, or subcutaneous site is another means to achieve in vivo expression.

In the relevant polynucleotides (e.g., expression vectors), the nucleic acid sequence encoding an anti-sense ARDAP RNA is operatively linked to a promoter or enhancer-promoter combination. Enhancers provide expression specificity in terms of time, location, and level. Unlike a promoter, an enhancer can function when located at variable distances from the transcription initiation site, provided a promoter is present. An enhancer can also be located downstream of the transcription initiation site.

Suitable expression vectors include plasmids and viral vectors such as herpes viruses, retroviruses, vaccinia viruses, attenuated vaccinia viruses, canary pox viruses, adenoviruses and adeno-associated viruses, among others.

Polynucleotides can be administered in a pharmaceutically acceptable carrier. Pharmaceutically acceptable carriers are biologically compatible vehicles that are suitable for administration to an animal or a human, e.g., physiological saline or liposomes. A therapeutically effective amount is an amount of the polynucleotide that is capable of producing a medically desirable result (e.g., a decreased ARDAP gene expression level or ARDAP protein activity level) in a treated subject. As is well known in the medical arts, the dosage for any one subject depends upon many factors, including the subject's size, body surface area, age, the particular compound to be administered, sex, time and route of administration, general health, and other drugs being administered concurrently. Dosages will vary, but a preferred dosage for administration of polynucleotide is from approximately 10⁶ to 10¹² copies of the polynucleotide molecule. This dose can be repeatedly administered, as needed. Routes of administration can be any of those listed above.

As a homolog to the human ARDAP gene, an animal ARDAP gene (e.g., the mouse ARDAP gene) can be used to generate a transgenic animal (e.g., a transgenic mouse) that serves as an animal model for developing drugs to treat cancer (e.g., breast cancer).

As used herein, "transgenic animal" includes the founder transgenic animals and progeny of the founders, as well as cells and tissues from such animals. Transgenic animals can be farm animals such as pigs, goats, sheep, cows, horses, and rabbits, rodents such as rats, guinea pigs, and mice, and non-human primates such as baboons, monkeys, and chimpanzees. Transgenic pigs and mice are particularly useful.

A transgenic animal of the invention contains a nucleic acid encoding an ARDAP protein integrated within its genome. As used herein, the term "ARDAP protein" refers to a wild-type ARDAP protein or a functionally equivalent variant, e.g., a fragment of the full-length protein.

It is contemplated that it may be useful to identify intron sequences, if any, in a genome and include them in a nucleic acid encoding an ARDAP protein.

In transgenic animals of the invention, the nucleic acid is operably linked to a regulatory element that can promote expression of the ARDAP gene, e.g., in breast. As used herein, the term "operably linked" refers to the placement of the regulatory element and nucleic acid in such a manner that the nucleic acid is transcribed. The regulatory element can be a breast-specific promoter, such as an MMTV-ras or MMTV-myc promoter.

The invention also features expression vectors suitable for generating transgenic animals of the invention. The expression vectors can include a promoter capable of mediating expression in breast operably linked to a nucleic acid encoding an ARDAP protein as described above.

Various techniques known in the art can be used to introduce expression vectors into animals to produce the founder lines of the transgenic animals. Such techniques include, but are not limited to, pronuclear microinjection (U.S Patent No. 4,873,191), retrovirus mediated gene transfer into germ lines (Van der Putten et al. (1985) Proc. Natl. Acad. Sci. USA 82, 6148), gene targeting into embryonic stem cells (Thompson et al. (1989) Cell 56, 313), electroporation of embryos (Lo, (1983) Mol. Cell. Biol. 3, 1803), and transformation of somatic cells in vitro followed by nuclear transplantation (Wilmut et al. (1997) Nature 385(6619), 810-813; and Wakayama et al. (1998) Nature 394, 369-374).
In one example, the expression vector is microinjected into an ovum or embryo of an animal or into embryonic stem cells of an animal.

Once transgenic animals have been generated (e.g., a transgenic mouse generated according to the Current Protocol (Wiley, USA) and Manipulate the Mouse Embryo (Hogan Beddington and Costantini Lacy, CSHL Press), expression of the ARDAP gene can be assessed using standard techniques. Initial screening can be accomplished by Southern blot analysis or PCR techniques to determine whether or not integration of the transgene has taken place. See, for example, Sambrook et al. (1989) Molecular Cloning, A Laboratory Manual, second edition, Cold Spring Harbor Press, Plainview, NY. Expression of the nucleic acid encoding an ARDAP protein in the tissues of the transgenic animals can be assessed using techniques that include, but are not limited to, Northern blot analysis of tissue samples obtained from the animal, in situ hybridization analysis, and reverse-transcriptase PCR (RT-PCR).

Transgenic animals of the invention can be used as cancer (e.g., breast cancer) models. In particular, these animals can be used to identify a compound or composition effective for treatment or prevention of cancer (e.g., breast cancer). Compounds or compositions can be identified by administering a test compound or composition to a transgenic animal of the invention or by contacting the test compound or composition with an organ, a tissue (e.g., breast) or cells (e.g., breast cells) derived from the transgenic animal. Effects of the test compound or composition on cancer (e.g., breast cancer) of the transgenic animal, organ, tissues or cells are evaluated. For example, the size of the cancer from clinical pathology identification can be assessed in the transgenic animals. Test compounds or compositions that palliate the cancer symptoms can be effective for treatment or prevention of cancer.

Test compounds can be formulated into pharmaceutical compositions by admixing the compounds with pharmaceutically acceptable non-toxic excipients or carriers and administered to transgenic animals of the invention by any route of administration. For example, parenteral routes such as subcutaneous, intramuscular, intravascular, intradermal, intranasal, inhalation, intrathecal, or intraperitoneal administration, and enteral routes such as sublingual, oral, or rectal administration can be used.

This invention also provides methods for diagnosing and treating breast cancer and identifying therapeutic compounds for treating breast cancer based on the finding that the androgen receptor level decreases in breast tumors.

A diagnostic method of this invention involves comparing the androgen receptor gene expression level or the androgen receptor protein activity level in a sample (e.g., a breast sample) prepared from a subject with that in a sample prepared from a normal person, i.e., a person who does not suffer from breast cancer. A lower androgen receptor gene expression or activity level indicates that the subject is suffering from or at risk for developing breast cancer. The methods of this invention can be used on their own or in conjunction with other procedures to diagnose breast cancer in appropriate subjects.

The androgen receptor gene expression level can be determined at either the mRNA level or at the protein level using methods similar to those described above. The androgen receptor protein activity can be determined by methods well known in the art, e.g., by measuring its ability to transactivate an androgrn-responsive gene. See, e.g., the Example below.

This invention also provides a method for identifying candidate compounds (e.g., proteins, peptides, peptidomimetics, peptoids, antibodies, or small molecules) that increase the androgen receptor gene expression level or androgen receptor protein activity level in a cell (e.g., a breast cell) using methods similar to those described above. Compounds thus identified can be used to treat breast cancer.

This invention also provides a method for preventing and treating breast cancer. Subjects to be treated can be identified, for example, by determining the androgen receptor gene expression level or the androgen receptor protein level in a sample prepared from a subject by methods described above. If the androgen receptor gene expression level or the androgen receptor protein level is lower in the sample from the subject than that in a sample from a normal person, the subject is a candidate for treatment with an effective amount of compound that modulates the androgen receptor level in the subject. The treatment method can be performed in vivo or ex vivo, alone or in conjunction with other drugs or therapy.

In one in vivo approach, a therapeutic composition (e.g., a composition containing a compound that increases the androgen receptor gene expression level or the androgen receptor protein activity level in a cell, a polynucleotide containing a nucleic acid sequence encoding an androgen receptor protein, or a androgen receptor protein itself) is administered to the subject using methods similar to those described above.

An ex vivo strategy for treating subjects with breast cancer associated with inadequate androgen receptor level can involve transfecting or transducing cells obtained from the subject with a polynucleotide encoding an androgen receptor protein. Alternatively, a cell can be transfected in vitro with a vector designed to insert, by homologous recombination, a new, active promoter upstream of the transcription start site of the naturally occurring endogenous androgen receptor gene in the cell's genome. Such methods, which "switch on" an otherwise largely silent gene, are well known in the art. After selection and expansion of a cell that expresses androgen receptor at a desired level, the transfected or transduced cells are then returned to the subject. The cells can be any of a wide range of types including, without limitation, neral cells, hemopoietic cells (e.g., bone marrow cells, macrophages, monocytes, dendritic cells, T cells, or B cells), fibroblasts, epithelial cells, endothelial cells, keratinocytes, or muscle cells. Such cells act as a source of the androgen receptor protein for as long as they survive in the subject.

The ex vivo methods include the steps of harvesting cells from a subject, culturing the cells, transducing them with an expression vector, and maintaining the cells under conditions suitable for expression of the androgen receptor gene. These methods are known in the art of molecular biology. The transduction step is accomplished by any standard means used for ex vivo gene therapy, including calcium phosphate, lipofection, electroporation, viral infection, and biolistic gene transfer. Alternatively, liposomes or polymeric microparticles can be used. Cells that have been successfully transduced can then be selected, for example, for expression of the androgen receptor gene. The cells may then be injected or implanted into the subject.

The specific example below is to be construed as merely illustrative, and not limitative of the remainder of the disclosure in any way whatsoever. Without further elaboration, it is believed that one skilled in the art can, based on the description herein, utilize the present invention to its fullest extent. All publications recited herein are hereby incorporated by reference in their entirety.

### EXAMPLE

The human ARDAP is located in the region of chromosome 1q13.3-21.

The human ARDAP mRNA levels were assessed in normal human tissues and in various tumor tissues using Northern blotting. ARDAP was detected only in human breast tumor, but not in normal breast, esophagus, thyroid, liver, brain, placenta, uterus, testis, lung, prostate, stomach, and heart tissues or esophagus, thyroid, liver, and brain tumor tissues.

Paired breast tumor and normal tissue adjacent to the tumor were isolated from breast cancer patients. Using Northern blotting, it was discovered that ARDAP mRNA is overly expressed in the tumor compared to the adjacent normal breast tissue. An ARDAP variant that is shorter in length was detected in some of the samples, suggesting that ARDAP may be alternatively spliced. In situ hybridization of ARDAP mRNA also indicates that ARDAP mRNA is abundant in tumor cells but rare in normal cells.

To determine cellular localization of the ARDAP protein, the ARDAP coding region was fused to GFP in an expression vector, and the vector transfected into human breast MCF-7 cells. The fusion protein was localized in the nucleus, indicating that ARDAP is a nuclear protein.

Contrary to ARDAP, AR was found to be under-expressed in breast tumors compared to normal breast tissues. A yeast two-hybrid system was used to determine whether ARDAP binds to AR. The interaction of AR and ARDAP in vivo was confirmed by this study. To further confirm that ARDAP binds to AR, ARDAP and AR were co-expressed as fusion proteins. Since immunoprecipitation of AR led to isolation of ARDAP and immunoprecipitation of ARDAP led to isolation of AR, the physical interaction of ARDAP and AR was confirmed.

To determine whether the physical association between the AR and ARDAP proteins is biochemically significant, the α-fetoprotein gene (AFP) promoter was used in a luciferase reporter construct. An enhancer region in the AFP promoter contains an androgen responsive element (ARE). A control isogenic luciferase reporter containing a mutated ARE was also used in the study to determine whether the ARE is responsible for mediating any biochemical effects.

MCF7 (breast cells) and G2 cells (hepatoma cells) were transfected with the wild type or control reporter and (1) mock DNA, (2) DNA encoding AR, (3) DNA encoding ARDAP, or (4) DNA encoding AR and DNA encoding ARDAP. Little change in luciferase activity was observed in all cells when the control reporter was used in combination with DNA (1)-(4), confirming that the mutant ARE is defective for AR-regulated transactivation. However, when the wild type reporter was used, luciferase activity increased about 6 times (relative to the control reporter) when AR was expressed. Little increase (about 30%, relative to the control reporter) in luciferase activity was observed when ARDAP was expressed. Surprisingly, the simultaneous expression of AR and ARDAP resulted in less increase (about 2 times, relative to the control reporter) in luciferase activity than when AR was expressed alone. This result indicated that ARDAP reduces the transactivation activity of AR on the AFP promoter.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claim.

## Claims

1. A pure polypeptide comprising an amino acid sequence at least 70% identical to SEQ ID NO: 4, wherein the polypeptide, when expressed in a cell, decreases transactivation of an androgen-responsive gene by an androgen receptor.

2. The polypeptide of claim 1, wherein the amino acid sequence is at least 80%, preferably 90% and more preferably 95% identical to SEQ ID NO: 4.

3. The polypeptide of claim 2, wherein the amino acid sequence is SEQ ID NO: 4.

4. An isolated nucleic acid **characterized in that** it hybridizes under stringent conditions to SEQ ID NO: 3 or a complementary sequence thereof.

5. The nucleic acid of claim 4, wherein the nucleic acid encodes a polypeptide of claim 1, wherein the amino acid sequence preferably is SEQ ID NO: 4.

6. The nucleic acid of claim 4, wherein the nucleic acid is SEQ ID NO: 3 or a complementary sequence thereof.

7. An antibody against the polypeptide of SEQ ID NO: 4.

8. A cell, preferably a breast cell, comprising a nucleic acid of claim 4, wherein the cell expresses the nucleic acid.

9. A method of expressing a transcript in a cell, preferably in a breast cell, the method comprising:
introducing a vector into said cell, the vector containing a nucleic acid encoding a transcript; and
expressing the transcript in the cell;
wherein the transcript is **characterized in that** it hybridizes under stringent conditions to SEQ ID NO: 3 or a complementary sequence thereof.

10. The method of claim 9, wherein the transcript encodes a polypeptide of claim 1.

11. A method of determining whether a subject is suffering from or at risk for developing breast cancer, the method comprising:
providing a sample, preferably a breast tissue sample, from a subject, the sample containing an androgen receptor down-regulatory-associated protein (i.e., ARDAP) gene product; and
determining an ARDAP gene expression level in the sample;
wherein the ARDAP gene expression level in the sample, if higher than that in a sample from a normal subject, indicates that the subject is suffering from or at risk for developing breast cancer.

12. A method of determining whether a subject is suffering from or at risk for developing breast cancer, the method comprising:
providing a sample, preferably a breast tissue sample, from a subject, the sample containing an ARDAP gene product; and
determining an ARDAP activity level in the sample;
wherein the ARDAP activity level in the sample, if higher than that in a sample from a normal subject, indicates that the subject is suffering from or at risk for developing breast cancer.

13. A method of identifying a compound for treating breast cancer, the method comprising:
contacting a compound with a cell, preferably a breast cell, expressing an ARDAP gene, and
determining an ARDAP gene expression level in the cell,
wherein the ARDAP gene expression level in the presence of the compound, if lower than that in the absence of the compound, indicates that the compound is a candidate for treating breast cancer.

14. A method of identifying a compound for treating breast cancer, the method comprising:
contacting a compound with a cell, preferably a breast cell, expressing an ARDAP gene, and
determining an ARDAP activity level in the cell,
wherein the ARDAP activity level in the presence of the compound, if lower than that in the absence of the compound, indicates that the compound is a candidate for treating breast cancer.

15. A method of treating breast cancer, the method comprising:
identifying a subject suffering from or being at risk for developing breast cancer, and
administering to the subject a composition, preferably into a breast cell, to decrease an ARDAP gene expression level in the subject.

16. The method of claim 15, wherein the composition includes a nucleic acid encoding a transcript, the transcript being **characterized in that** it hybridizes under stringent conditions to SEQ ID NO:3.

17. A method of treating breast cancer, the method comprising:
identifying a subject suffering from or being at risk for developing breast cancer, and
administering to the subject a composition, preferably into a breast cell, to decrease an ARDAP activity level in the subject.

18. The method of claim 17, wherein the composition contains an antibody of claim 7.

19. A pharmaceutical composition comprising a pharmaceutically acceptable carrier and a nucleic acid encoding a transcript, wherein the transcript is **characterized in that** it hybridizes under stringent conditions to SEQ ID NO: 3 or an antibody of claim 7.

20. A transgenic animal whose genome comprises a transgene containing a nucleic acid encoding a polypeptide of claim 1, wherein the transgenic animal exhibits a decreased androgen response as compared to a wild-type animal.

21. The transgenic animal of claim 20, wherein the animal is a rodent, preferably a mouse.

22. The transgenic animal of claim 20, wherein the amino acid sequence is SEQ ID NO:4.

23. The transgenic animal of claim 22, wherein the animal is a rodent, preferably a mouse.

24. A method of determining whether a subject is suffering from or at risk for developing breast cancer, the method comprising:
providing a sample, preferably a breast tissue sample, from a subject, the sample containing an androgen receptor gene product; and
determining an androgen receptor gene expression level in the sample;
wherein the androgen receptor gene expression level in the sample, if lower than that in a sample from a normal subject, indicates that the subject is suffering from or at risk for developing breast cancer.

25. A method of determining whether a subject is suffering from or at risk for developing breast cancer, the method comprising:
providing a sample, preferably a breast tissue sample, from a subject, the sample containing an androgen receptor gene product; and
determining an androgen receptor activity level in the sample;
wherein the androgen receptor activity level in the sample, if lower than that in a sample from a normal subject, indicates that the subject is suffering from or at risk for developing breast cancer.

26. A method of identifying a compound for treating breast cancer, the method comprising:
contacting a compound with a cell, preferably a breast cell, expressing an androgen receptor gene, and
determining an androgen receptor gene expression level in the cell,
wherein the androgen receptor gene expression level in the presence of the compound, if higher than that in the absence of the compound, indicates that the compound is a candidate for treating breast cancer.

27. A method of identifying a compound for treating breast cancer, the method comprising:
contacting a compound with a cell, preferably a breast cell, expressing an androgen receptor gene, and
determining an androgen receptor activity level in the cell,
wherein the androgen receptor activity level in the presence of the compound, if higher than that in the absence of the compound, indicates that the compound is a candidate for treating breast cancer.

28. A method of treating breast cancer, the method comprising:
identifying a subject suffering from or being at risk for developing breast cancer, and
administering to the subject a composition, preferably into a breast cell, to increase an androgen receptor gene expression level in the subject.

29. The method of claim 28, wherein the composition includes a nucleic acid encoding an androgen receptor protein.

30. The method of claim 29, wherein the composition is administered into a breast cell.

31. The method of claim 28, wherein the composition includes an androgen receptor protein.

32. The method of claim 31, wherein the composition is administered into a breast cell.

33. A method of treating breast cancer, the method comprising:
identifying a subject suffering from or being at risk for developing breast cancer, and
administering to the subject a composition, preferably into a breast cell, to increase an androgen receptor activity level in the subject.
